(19) Europäisches Patentamt
European Patent Office
Office européen des brevets

(11) **EP 3 723 095 B1**

(12) **EUROPEAN PATENT SPECIFICATION**

(45) Date of publication and mention
of the grant of the patent:
**28.02.2024 Bulletin 2024/09**

(51) International Patent Classification (IPC):
**G16B 15/30** (2019.01)    **G16H 70/40** (2018.01)
**G16C 20/30** (2019.01)    **G16C 20/70** (2019.01)

(21) Application number: **20163195.9**

(52) Cooperative Patent Classification (CPC):
**G16H 70/40; G16B 15/30; G16C 20/30;**
**G16C 20/70**

(22) Date of filing: **13.03.2020**

(54) **LATENT SPACE EXPLORATION USING LINEAR-SPHERICAL INTERPOLATION REGION METHOD**

EXPLORATION EINES LATENTEN RAUMS UNTER VERWENDUNG EINES VERFAHRENS FÜR LINEARE-SPHÄRISCHE INTERPOLATIONSREGION

EXPLORATION D'UN ESPACE LATENT À L'AIDE D'UN PROCÉDÉ DE RÉGION D'INTERPOLATION LINÉAIRE-SPHÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.04.2019 US 201962832489 P**
**19.06.2019 US 201916445811**

(43) Date of publication of application:
**14.10.2020 Bulletin 2020/42**

(73) Proprietor: **Accenture Global Solutions Limited Dublin 4 (IE)**

(72) Inventors:
 • **AIN, Qurrat Ul**
 **DUBLIN, 4 (IE)**
 • **McCARTHY, Nicholas**
 **DUBLIN, 4 (IE)**
 • **HAYES, Jeremiah**
 **DUBLIN, 4 (IE)**
 • **O'KELLY, Philip**
 **DUBLIN, 4 (IE)**
 • **MOREAU, Patrick**
 **DUBLIN, 4 (IE)**

(74) Representative: **Boult Wade Tennant LLP Salisbury Square House 8 Salisbury Square London EC4Y 8AP (GB)**

(56) References cited:
**EP-A1- 3 869 513    US-A1- 2017 161 635**

 • **RAFAEL GÓMEZ-BOMBARELLI ET AL: "Automatic chemical design using a data-driven continuous representation of molecules", ARXIV.ORG, CORNELL UNIVERSITY LIBRARY, 201 OLIN LIBRARY CORNELL UNIVERSITY ITHACA, NY 14853, 7 October 2016 (2016-10-07), XP081350646, DOI: 10.1021/ACSCENTSCI.7B00572**
 • **SAMANTA B ET AL: "Designing Random Graph Models Using Variational Autoencoders With Applications to Chemical Design", ARXIV.ORG, CORNELL UNIVERSITY LIBRARY, 201 OLIN LIBRARY CORNELL UNIVERSITY ITHACA, NY 14853, 14 February 2018 (2018-02-14), XP080856702,**
 • **KUSNER M J ET AL: "Grammar variational autoencoder", arXiv:1703.01925v1, 6 March 2017 (2017-03-06), XP055550975, Retrieved from the Internet: URL:https://arxiv.org/abs/1703.01925v1 [retrieved on 2019-02-01]**
 • **CHANG D T: "Probabilistic Generative Deep Learning for Molecular Design", ARXIV.ORG, CORNELL UNIVERSITY LIBRARY, 201 OLIN LIBRARY CORNELL UNIVERSITY ITHACA, NY 14853, 11 February 2019 (2019-02-11), XP081028929,**
 • **ELTON D C ET AL: "Deep learning for molecular generation and optimization - a review of the state of the art", ARXIV.ORG, CORNELL UNIVERSITY LIBRARY, 201 OLIN LIBRARY CORNELL UNIVERSITY ITHACA, NY 14853, 11 March 2019 (2019-03-11), XP081132028,**

EP 3 723 095 B1

- **WHITE T: "Sampling Generative Networks", , 6 December 2016 (2016-12-06), XP055690302, Retrieved from the Internet: URL:https://arxiv.org/pdf/1609.04468.pdf [retrieved on 2020-04-29]**

**Description**

**FIELD OF THE INVENTION**

The present disclosure relates in general to the field of latent space exploration, and in particular methods and systems for identifying drug compounds for experimental usage in the treatment of diseases using latent space generated by variational auto-encoders based on the combination of drug molecular-structure data and drug biological-treatment data.

**BACKGROUND**

Basic techniques and equipment for ranking drug compounds, scoring gene expressions and enrichment pathways, and selecting predictive biomarkers are known in the art. Both drug data and biological data have features that can be described as discrete values using variational auto-encoders to generate latent spaces for modeling the probability distributions of latent variables that may be explored using interpolation methods and quantitative structure-activity relationship models. While various technologies have used either drug data or biological data independently to generate latent space, a multi-modal latent space based on the combination of drug molecular-structure data and biological-treatment data is desired to more efficiently identify optimal and/or new drug compounds for the treatment of diseases.
In "Automatic chemical design using a data-driven continuous representation of molecules", by Rafael Gomez-Bombarelli et al, there is described a method to convert discrete representations of molecules to and from a continuous representation.

**BRIEF SUMMARY**

The invention is set out in the appended claims.
The present disclosure may be embodied in various forms, including without limitation a system, a method or a computer-readable medium for latent space exploration using regional interpolation and quantitative structure-activity relationship (QSAR) models to navigate through a latent space generated from variational auto-encoders (VAE). The latent space may graphically represent a probability distribution of latent attributes associated with a plurality of drug compounds. Such latent attributes may comprise "hidden" data that would not have otherwise been observed and considered, without the use of the present disclosure. These latent attributes may be used to identify candidate compounds or molecules that may be used as new drugs to treat various diseases. In order to determine a list of optimal candidates, a regional interpolation method and a QSAR model may be utilized to determine an optimal path between two clusters of nodes in the latent space. Each node in the latent space may correspond to an embedding vector representing the probability distributions for the various attributes of one drug compound. The optimal path includes the list of embedding vectors comprising the top-ranked probability distributions for desired attributes associated with the treatment of certain diseases.
The regional interpolation method utilized to determine the optimal path in the latent space comprises a linear interpolation and a non-linear interpolation, such as spherical interpolation, circular interpolation, or elliptical interpolation. While the clusters of nodes may represent a region of interest in the latent space corresponding to the patent attributes for drug compounds known to be associated with the biological-treatment data for certain diseases, the optimal path of nodes may correspond to latent attributes that may have not yet been considered in the selection of drug compounds for the treatment of such diseases. The optimal path between two clusters may represent candidates for drug formulations, which may comprise either pre-existing or new compounds, that may be effective against the diseases associated with the two clusters. The targeted clusters are identified by a query performed on the entire set of vectors embedded in the latent space. The query may include a drug molecular-structure query, a drug treatment query, and/or a drug effect query. A cluster of nodes in the latent space identified by the query may be annotated or marked with a disease label that corresponds to a certain disease. In an example, the labelled nodes may represent drug compounds known to be effective in the treatment of the disease.
In some examples, a latent space may be generated via variational auto-encoders based on pre-existing drug data and human biological data for a plurality of drug compounds. This "input" data may include structural information for the drug compounds, as well as data regarding the effectiveness of the drug compounds against certain diseases. The structural information for the drug compounds may comprise simplified molecular-input line-entry system (SMILES) strings. In an example, the biological data may include datasets of genetic variation data, somatic mutation data, electronic health records, pathway enrichment data, gene expression data, protein expression data, disease ontology data, protein interactions data, and/or various scores/ranking associated with the drug compounds. The input data associated with each drug compound may be represented as an array or a vector. An input vector or array may comprise structured data, a dataset, a mathematical object, or a list of values that represent the drug data and biological data for a drug compound. The input vector represents a combination of the drug molecular-structure data and the drug biological-treatment data for a drug compound.

The variational auto-encoder may compress the input vectors for each drug compound based on attributes or correlations determined from the data during training. The output of a variational auto-encoder may describe the probability distributions, such multivariate Gaussian distributions, for latent attributes of drug compounds. The latent distributions are represented as a latent space, which comprises embedding or encoding vectors that describe the probability distributions for a plurality of drug compounds. In an examples, the elements of an embedding vector may represent the probability distributions for the latent attributes for a drug compound. A decoder may randomly sample from the probability distributions for desired attributes, and generate reconstruction vectors that may comprise structured data in a form similar to that of the input vectors that may be utilized to identify candidate drug compounds.

In certain examples, the aforementioned interpolation methods may be used to explore the latent space to determine and define the boundaries of an interpolation region to be further analyzed in order to identify candidate drug compounds. In some examples, the interpolation space or region of interest may be identified based on linear and spherical interpolation paths determined using the interpolation methods. The rankings of the compounds within each interpolation region are determined using a QSAR model based on the embedding vectors of the drug compounds and a biomarker. In some examples, the rankings of the drug compounds may be based on a predicted target-value for the compounds, such as a binding activity, toxicity, and/or efficacy value. A vector path between the two clusters is determined based on the ranking of the compounds within each interpolation region.

In an example, the vector path may represent prime compounds that may be optimal candidates for experimental usage in treating certain diseases. Further, in accordance with some examples, the benefits of this disclosure may include the discovery of new molecular formulation for existing drugs, and a reduction in the time spent during experimental testing by identifying optimal outputs. Examples of the present disclosure may enable a system/platform where a user may input their drug data and receive drug variations to test.

## BRIEF DESCRIPTION OF THE DRAWINGS

The foregoing and other objects, features, and advantages for embodiments of the present disclosure will be apparent from the following more particular description of the embodiments as illustrated in the accompanying drawings, in which reference characters refer to the same parts throughout the various views. The drawings are not necessarily to scale, emphasis instead being placed upon illustrating principles of the present disclosure.

Figures 1(a)-(g) are exemplary graphs illustrating latent space exploration at certain steps of an embodiment for determining a vector path between two clusters of probability distributions for drug compounds associated with the biological-treatment of diseases, in accordance with certain embodiments of the present disclosure.

Figure 2 is a block diagram illustrating an embodiment of a system for implementing latent space exploration, in accordance with certain embodiments of the present disclosure.

Figure 3 is a block diagram illustrating an embodiment of a computer architecture for a computer device for implementing latent space exploration, in accordance with certain embodiments of the present disclosure.

Figure 4 is a flowchart illustrating the use of latent space exploration to identify a new molecule that may potentially be used as new drug, in accordance with certain embodiments of the present disclosure.

Figure 5 is a flowchart illustrating steps of an embodiment for identifying top-ranked molecules that may potentially be used for experimental usage in the treatment of diseases, in accordance with certain embodiments of the present disclosure.

Figure 6 is a flowchart illustrating steps of an embodiment for determining a prime drug compound for the treatment of a disease, in accordance with certain embodiments of the present disclosure.

## DETAILED DESCRIPTION OF EMBODIMENTS OF THE INVENTION

Reference will now be made in detail to the embodiments of the present disclosure, examples of which are illustrated in the accompanying drawings.

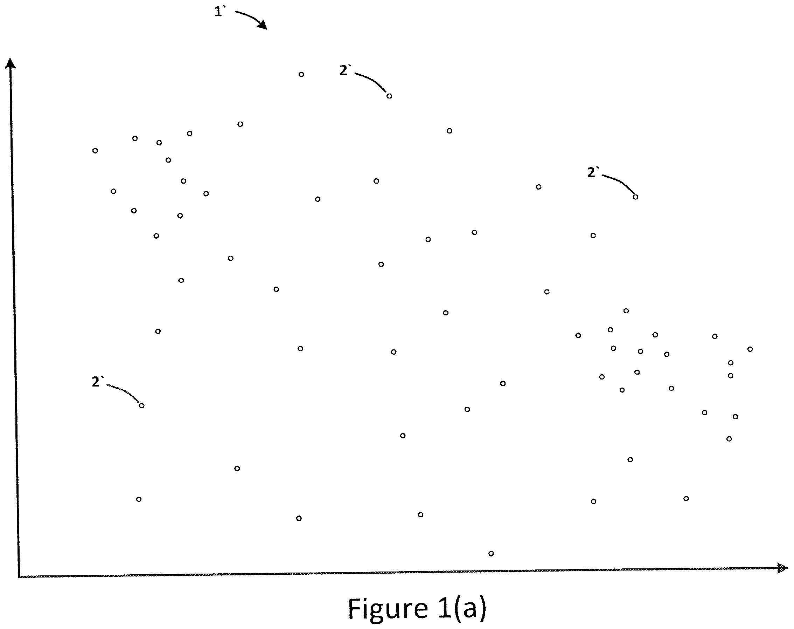

The present disclosure may be embodied in various forms, including a computer-implemented method, a product comprising a machine-readable medium, or a system for latent space exploration of a dataset based on drug molecular-structure data and drug biological-treatment data for a set of drug compounds in order to identify drug compounds having desired properties for treating diseases. A latent space **1** may be represented as a graphical plot of embedding vectors **2** for probability distributions of certain properties or attributes for the set of drug compounds **3**, in accordance with certain embodiments. **Figures 1(a)-(g)** illustrate exemplary graphs representing the exploration of a latent space **1**, in accordance with certain embodiments. **Figure 1(a)** illustrates an exemplary graph **1'** corresponding to the latent space **1**, wherein each node **2'** shown in the graph **1'** may correspond to an embedding vector **2** of the latent space **1**.

Embedding vectors **2** may be generated using a variational auto-encoder (VAE) **4** based on input data **5** representative of the drug compounds **3**. In an embodiment, an input vector **5** may be based on a combination of molecular-structure data **6** and biological-treatment data **7** corresponding to that drug compound **3**. For example, SMILES strings **6** and biological data **7** may be converted into vector representations that may be combined to generate embedding vectors **2**. The variational auto-encoder **4** may be trained so that the embedding vectors **2** map, or correlate, to the input vectors **5**.

Computations may be performed on the embedding vectors **2** in the latent space **1**, such as regional interpolation **8** and the decoding of random vectors **2** that are likely to correspond to desired attributes **9** for drug compounds **3**. In some embodiments, the latent space **1** comprises embedding vectors **2** describing probability distributions **10** for latent attributes **9** of drug compounds **3** having certain molecular-structure data **6** and certain biological-treatment data **7** that relate to certain diseases **11** to be treated. In an embodiment, embedding vectors **2** corresponding to drug compounds **3** having a predetermined biomarker **12** of interest may be targeted. Embedding vectors **2** may be targeted based on certain values **13** for the attributes **9**, such as a predetermined binding activity, toxicity, or efficacy value for a drug compound **3**. The embedding vectors **2** may be ranked based on such target values **13**, and decoded to identify the top-ranked drug compounds **3** for further experimental testing in laboratories **14**.

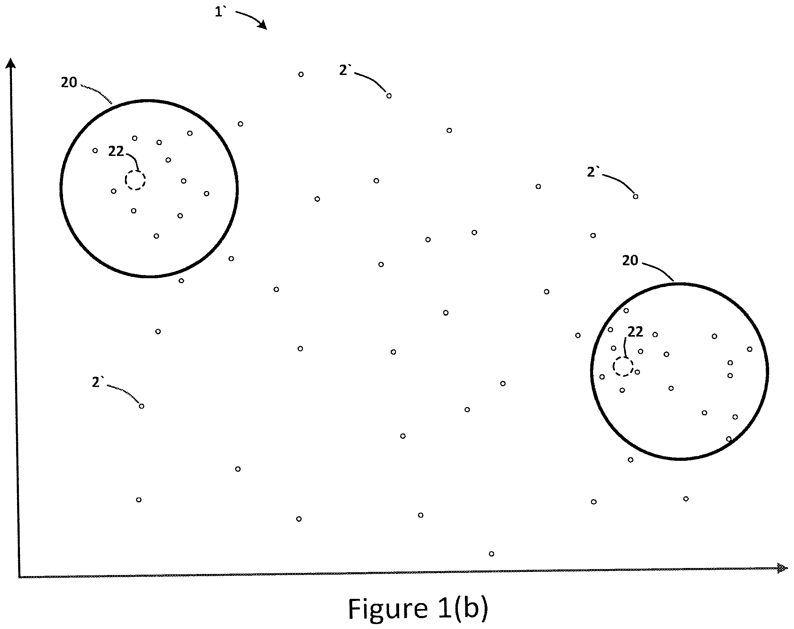

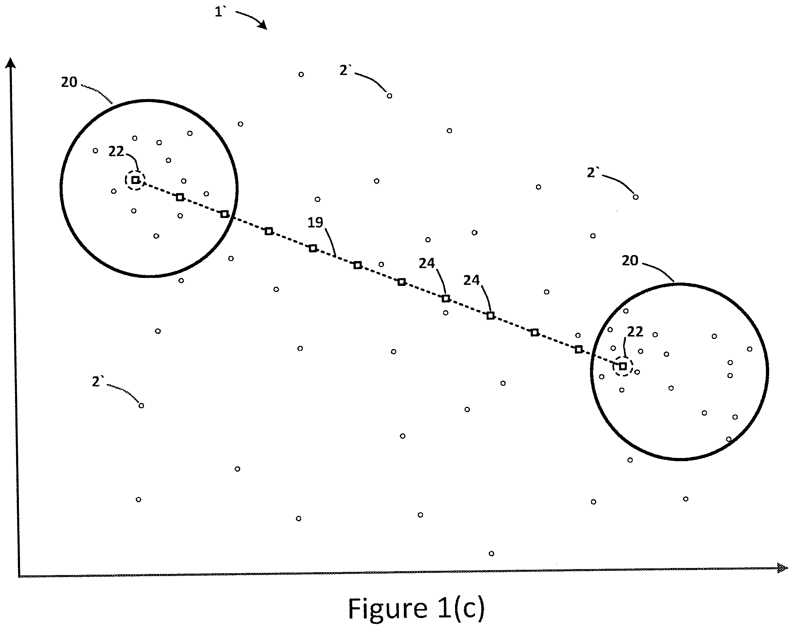

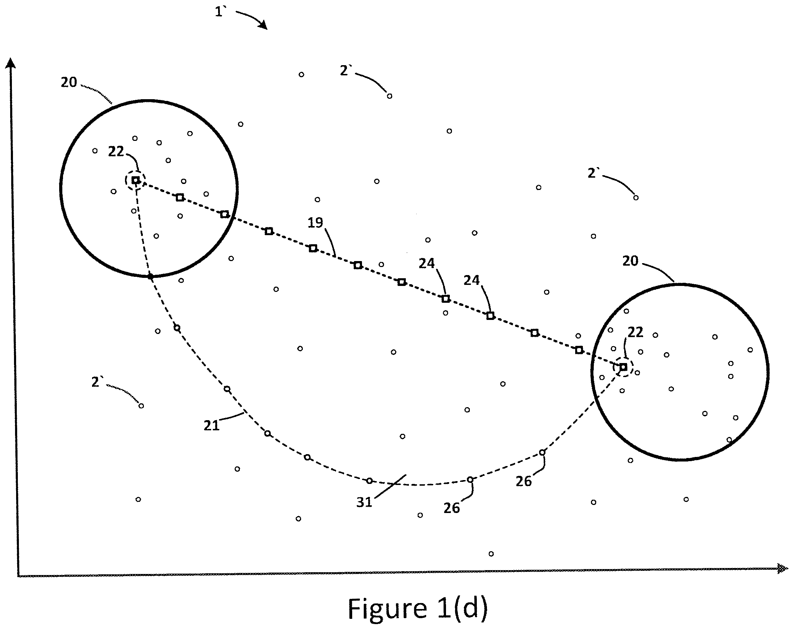

In an embodiment, a method may include an initial step of receiving drug molecular-structure data **6** and drug biological-treatment data **7** from various databases. Such received data **15** may be combined into a dataset **5** (*e.g.*, an input vector **5**) that may be converted via a variational auto-encoder **4** into an embedding dataset **2** (*e.g.*, an embedding vector **2**) represented in a latent space **1**. In accordance with some embodiments, the method may include the step of receiving a drug molecular-structure query **16**, a drug treatment query **17**, and a drug effect query **18**. The method may include the step of determining a linear interpolation path **19** between clusters **20** of embedding vectors **2** in the latent space **1**. **Figure 1(b)** illustrates such clusters **20**, and **Figure 1(c)** depicts an exemplary linear interpolation path **19**. The method may include the step of determining a curved or non-linear interpolation path **21** (such as a spherical, circular or elliptical path **21**) between such clusters **20** in the latent space **1**, as shown in **Figure 1(d)**.

In accordance with some embodiments, the determination of the linear interpolation path **19** and the determination of the non-linear interpolation path **21** may be based on one or more queries **16-18**. For example, the queries may comprise the drug molecular-structure query **16**, the drug treatment query **17**, and the drug effect query **18**. The targeted clusters **20** of embedding vectors **2** may have probability distributions **10** for attributes **9** of drug compounds **3** greater than a predetermined value, such that the clusters **20** of embedding vectors **2** are determined to be responsive to the drug molecular-structure query 16, the drug treatment query **17**, and/or the drug effect query **18**. In order words, the targeted clusters **20** comprise a region of embedding vectors **2** having a high probability for a desired attribute **9** that corresponds to a query **16-18**. In an embodiment, the interpolation paths **19** and **21** may extend from the centroid **22** of a first cluster **20** to the centroid **22** of a second cluster **20**, as shown in **Figure 1(d)**. Accordingly, the targeted clusters **20** in the latent space **1** may represent drug compounds **3** that correspond to two diseases **11** to be treated. Such diseases **11** may be targeted or predetermined based on the drug molecular-structure query **16**, the drug treatment query **17**, and/or the drug effect query **18**.

In certain embodiments, clusters in the latent space **1** may correspond to probability distributions **10** for attributes **9** of drug compounds **3** that may be associated with biological-treatment data **7** for specific diseases **11**. In an embodiment, the method may include the step of annotating or marking the latent space **1** with disease labels **23**. The disease labels **23** may correspond to diseases **11** that may be effectively treated by the drug compounds **3** represented by the corresponding clusters **20** in the latent space **1**. The clusters **20** of embedding vectors **2** may be assigned to certain diseases **11**, such as HIV or breast cancer.

In some embodiments, the method may include the steps of determining a first set of candidate points **24** on the linear interpolation path **19** based on a first predetermined stop-parameter **25**. The method may include the step of determining a second set of candidate points **26** on the non-linear (*e.g.* spherical, circular or elliptical) interpolation path **21** based on the first predetermined stop-parameter **25**. Accordingly, the two interpolation paths **19** and **21** may extend between the same two start and end points, *e.g.* the centroids **22** of the two clusters **20**. **Figure 1(d)** depicts the two sets of candidate points **24** and **26** on the two interpolation paths **19** and **21**, extending between two centroids **22**.

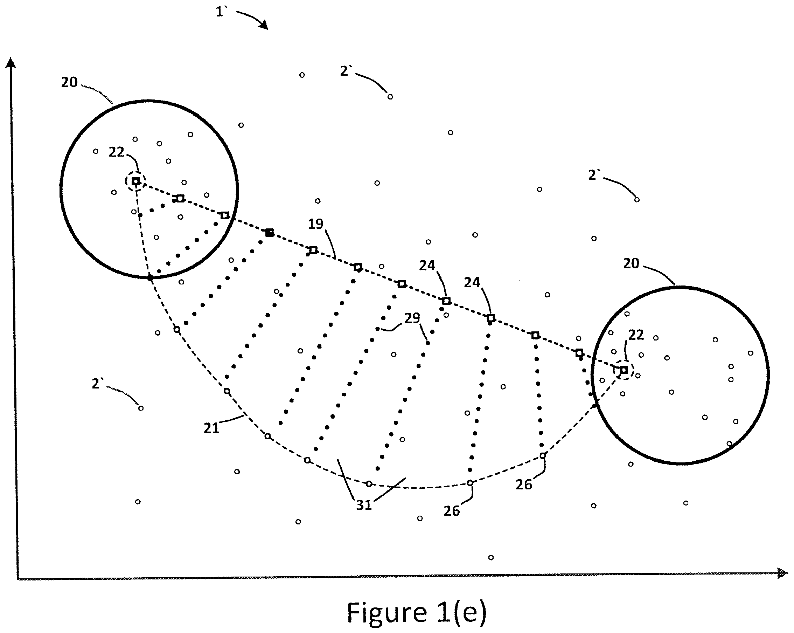

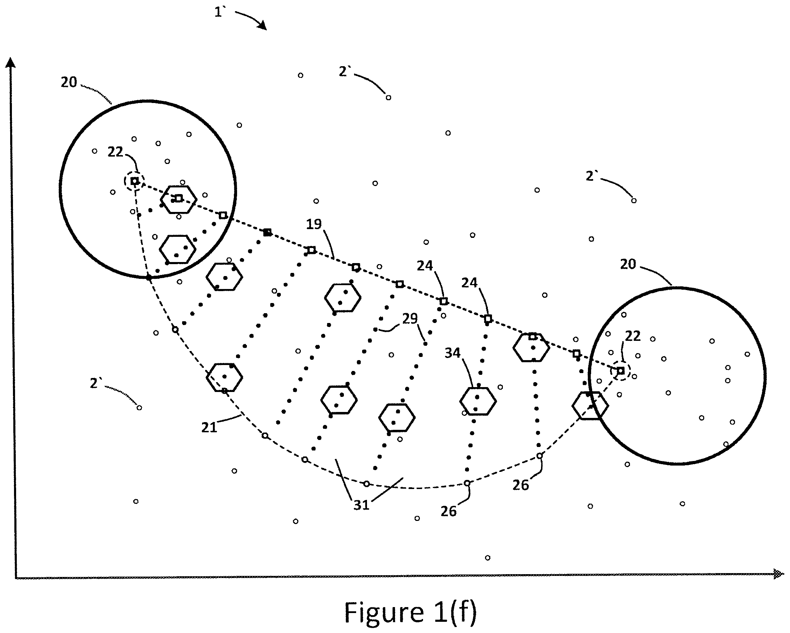

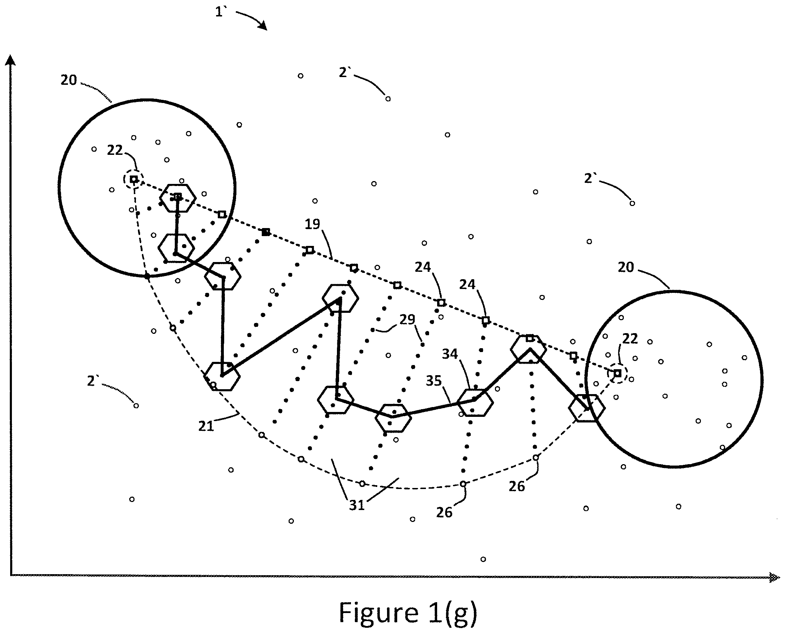

In certain embodiments, the method may further include the step of determining a linear chord interpolation path **28** between each candidate point **24** on the linear interpolation path **19** and each corresponding candidate point **25** on the non-linear interpolation path **21**. The method may include the steps of determining a third set of candidate points **29** on each linear chord interpolation path **28** based on a second predetermined stop-parameter **30**, and the step of determining an interpolation region **31** bound by the interpolation paths **19** and **21**. **Figure 1(e)** illustrates the candidate points **29** for the linear chord interpolation paths **28** within the interpolation region **31**. The method may determine a drug effect score **32** of each of the first set of candidate points **24**, each of the second set of candidate points **26**, and each of the third set of candidate points **29** using a quantitative structure-activity relationship (QSAR) model **33**. In accordance with certain embodiments, as shown in **Figure 1(f)**, the method may further include the step of determining prime or optimal candidate points **34** on each linear chord interpolation path **28** based on the drug effect scores **32**. **Figure 1(g)** illustrates a vector path **35** within the interpolation region **31** based on the rankings of the third set of candidate points **29**. Such ranking may be based on a comparison of the drug effect scores **32**. In an embodiment, the method may include the step of determining the optimal or prime drug molecular-structures **36** based on the prime candidate points **34**.

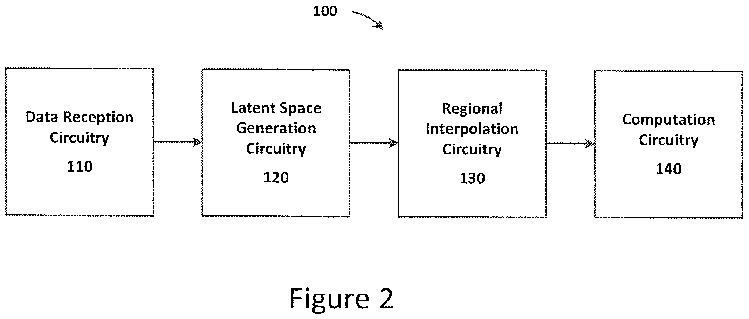

**Figure 2** illustrates an embodiment of a system **100** for implementing latent space exploration. The circuitry described herein may include the hardware, software, middleware, application program interfaces (APIs), and/or other components for implementing the corresponding features of the circuitry. Initially, a data reception circuitry **110** may be configured to receive drug molecular-structure data **6** and drug biological-treatment data **7**. In an embodiment, a latent space generation circuitry **120** may be configured to combine the received molecular-structure data **6** and the received biological-treatment data **7** into a combined dataset **5**, *e.g.* an input vector **5**, represented in a latent space **1** using a variational auto-encoder **4**. The latent space generation circuitry **120** may compress the input data **5** by applying convolutional neural-network layers and encoder models, *e.g.* variational auto-encoders (VAEs) **4** models, and generate embedding vectors **2** that represent probability distributions **10** for latent attributes **9**. Accordingly, the latent space generation circuitry **120** may construct a latent space **1** from the received information, in accordance with certain embodiments. The resulting graphical representation **1'** may illustrate structured data having a specific format where each point or node **2'** may represent probability distributions **10** for latent attributes **9** that may correspond to a drug compound **3**.

The latent space exploration system 100 may further include a regional interpolation circuitry **130** that may be configured to: determine a linear interpolation path **19** between clusters **20** of embedding vectors **2** in the latent space **1**; determine a curved or non-linear (*e.g.*, spherical, circular or elliptical) interpolation path **21** between clusters **20** of embedding vectors **2** in the latent space **1**; determine a first set of candidate points **24** on the linear interpolation path **19** based on a first predetermined stop-parameter **25**; determine a second set of candidate points **26** on the non-linear interpolation path **21** based on the first predetermined stop-parameter **25**; determine a linear chord interpolation path **28** between each candidate point **24** on the linear interpolation path **19** and each corresponding candidate point **26** on the non-linear interpolation path **21**; and, determine a third set of candidate points **29** on each linear chord interpolation path **28** based on a second predetermined stop-parameter **30**. The regional interpolation circuitry **130** may determine an interpolation region bound **31** by the interpolation paths **19** and **21**.

In some embodiments, the system may include a computation circuitry **140** configured to apply a QSAR model **33** to the embedding vectors **2** in an interpolation region **31** of the latent space **1**. The computation circuitry **140** may determine a drug effect score **32** of each of the first plurality of candidate points **24**, each of the second plurality of candidate points **26**, and each of the third plurality of candidate points **29** using the quantitative structure-activity relationship model **33**. The computation circuitry **140** may further determine prime candidate points **34** based on the drug effect scores **32**, and determine prime drug molecular structures **36** based on the prime candidate points **34**. Overall, executing the latent space exploration process provides improvements to the computing capabilities of a computer device executing the process by reducing the search space and by allowing for more efficient data analysis in order to analyze large amounts of data in a shorter amount of time.

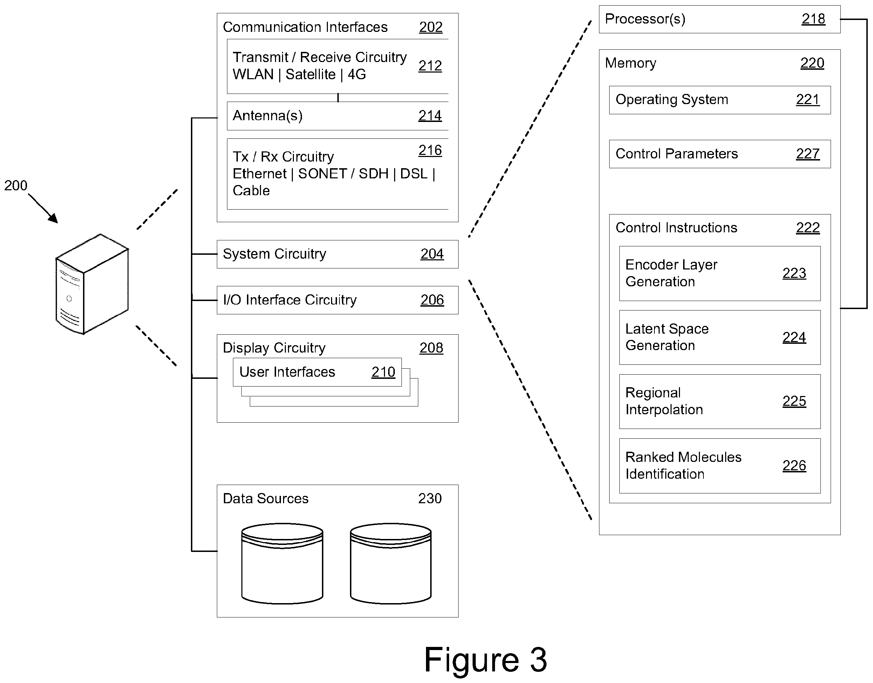

**Figure 3** illustrates an exemplary computer architecture of a computer device **200** on which the features of the latent space exploration system **100** may be executed. The computer device **200** includes communication interfaces **202**, system circuitry **204**, input/output (I/O) interface circuitry **206**, and display circuitry **208**. The graphical user interfaces (GUIs) **210** displayed by the display circuitry **208** may be representative of GUIs generated by the system **100** to present a query to an enterprise application or end user, requesting information on a compound **3** to be replaced and/or compound attributes desired to be satisfied by a candidate discovery compound. The graphical user interfaces (GUIs) **210** displayed by the display circuitry **208** may also be representative of GUIs generated by the system **100** to receive query inputs **16-18** identifying the compound **3** to be replace and/or compound attributes **9** desired to be satisfied by a candidate discovery compound. The GUIs **210** may be displayed locally using the display circuitry **208**, or for remote visualization, *e.g.*, as HTML, JavaScript, audio, and video output for a web browser running on a local or remote machine. Among other interface features, the GUIs **210** may further render displays of any new formulations resulting from the replacement of compounds(s) **3** with discovery compound(s) selected from the processes described herein.

The GUIs **210** and the I/O interface circuitry **206** may include touch sensitive displays, voice or facial recognition inputs, buttons, switches, speakers and other user interface elements. Additional examples of the I/O interface circuitry **206** includes microphones, video and still image cameras, headset and microphone input / output jacks, Universal Serial Bus (USB) connectors, memory card slots, and other types of inputs. The I/O interface circuitry **206** may further include magnetic or optical media interfaces (e.g., a CDROM or DVD drive), serial and parallel bus interfaces, and keyboard and mouse interfaces.

The communication interfaces **202** may include wireless transmitters and receivers ("transceivers") **212** and any antennas **214** used by the transmit-and-receive circuitry of the transceivers **212**. The transceivers **212** and antennas **214** may support WiFi network communications, for instance, under any version of IEEE 802.11, *e.g.*, 802.11n or 802.11ac, or other wireless protocols such as Bluetooth, Wi-Fi, WLAN, cellular (4G, LTE/A). The communication interfaces **202** may also include serial interfaces, such as universal serial bus (USB), serial ATA, IEEE 1394, lighting port,

I$^2$C, slimBus, or other serial interfaces. The communication interfaces **202** may also include wireline transceivers **216** to support wired communication protocols. The wireline transceivers **216** may provide physical layer interfaces for any of a wide range of communication protocols, such as any type of Ethernet, Gigabit Ethernet, optical networking protocols, data over cable service interface specification (DOCSIS), digital subscriber line (DSL), Synchronous Optical Network (SONET), or other protocol.

The system circuitry **204** may include any combination of hardware, software, firmware, APIs, and/or other circuitry. The system circuitry **204** may be implemented, for example, with one or more systems on a chip (SoC), application specific integrated circuits (ASIC), microprocessors, discrete analog and digital circuits, and other circuitry. The system circuitry **204** may implement any desired functionality of the system **100**. As just one example, the system circuitry **204** may include one or more instruction processor **218** and memory **220**.

The memory **220** stores, for example, control instructions **222** for executing the features of the system **100**, as well as an operating system **221**. In one implementation, the processor **218** executes the control instructions **222** and the operating system **221** to carry out any desired functionality for the system **100**, including those attributed to encoder layer generation **223** and latent space generation **224** (*e.g.*, relating to the latent space generation circuitry **120**), regional interpolation **225** (*e.g.*, relating to the regional interpolation circuitry **130**), and/or ranked molecules identification **226** (*e.g.*, relating to the computation circuitry **140**). The control parameters **227** provide and specify configuration and operating options for the control instructions **222**, operating system **221**, and other functionality of the computer device **200**.

The computer device **200** may further include various data sources **230**. Each of the databases that are included in the data sources **230** may be accessed by the system **100** to obtain data for consideration during any one or more of the processes described herein. For example, the data reception circuitry **110** may access the data sources **230** to receive the input data for generating the latent space **1**.

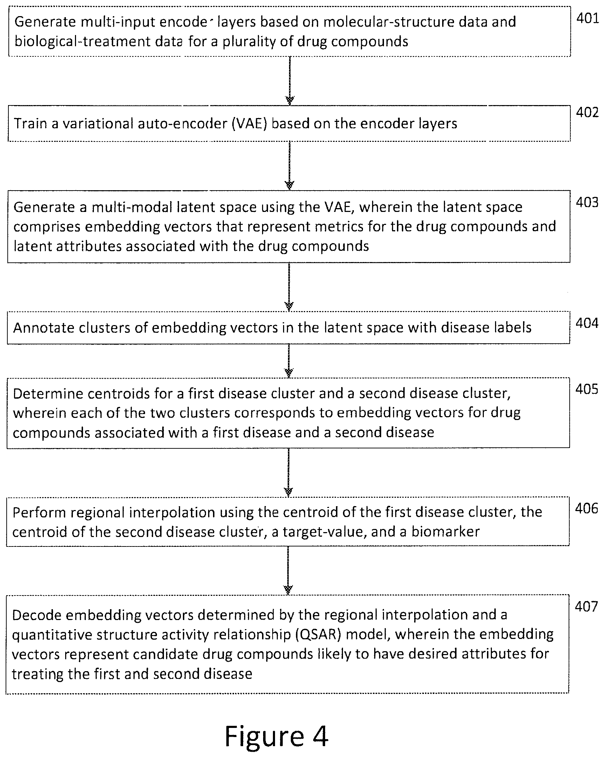

**Figures 4-6** show flowcharts representing exemplary processes or methods implemented by the system **100**, in accordance with certain embodiments. The processes may be implemented by a computing device **200**, system, and/or circuitry components as described herein.

In an embodiment, as set forth in block **401** of **Figure 4**, a system **100** may generate multi-input encoder layers **37** based on molecular-structure data **6** and biological-treatment data **7** for a plurality of drug compounds **3**. As noted above, this step for encoder layer generation **223** may be implemented by the latent space generation circuitry **120**. The system **100** may further train a variational auto-encoder (VAE) **4** based on the encoder layers **37** (block **402**), and generate a multi-modal latent space **1** using the variational auto-encoder **4** (block **403**). The latent space **1** may comprise embedding vectors **2** that represent probability distributions **10** for latent attributes **9** associated with the drug compounds **3**. In some embodiments, as depicted by block **404**, the system **100** may annotate clusters **20** of embedding vectors **2** in the latent space **1** with disease labels **23**. The system **100** may also determine centroids **22** for a first disease cluster **20** and a second disease cluster **20**, wherein each of the two clusters **20** corresponds to embedding vectors **2** for drug compounds **3** associated with a first disease **11** and a second disease **11**, as set forth in block 405. In an embodiment, the first disease **11** may be associated with HIV and the second disease **11** may be associated with breast cancer.

In addition, the system **100** may perform regional interpolation (block **406**) using the centroid **22** of the first disease cluster **20**, the centroid **22** of the second disease cluster **20**, a target-value **13**, and a biomarker **12**. This step for regional interpolation **225** may be implemented by the regional interpolation circuitry **130**. In an embodiment, the target-value **13** may comprise a binding activity value greater than the value of 5. In some embodiments, the biomarker **12** may represent a high gene expression in genes related to breast cancer or inflammation. As depicted in block **407**, via the computation circuitry 140, a system **100** may decode embedding vectors **2** determined by the regional interpolation and a quantitative structure activity relationship (QSAR) model **33**, wherein the embedding vectors **2** represent candidate drug compounds **3** likely to have desired attributes **9** for treating the first and second disease **11**.

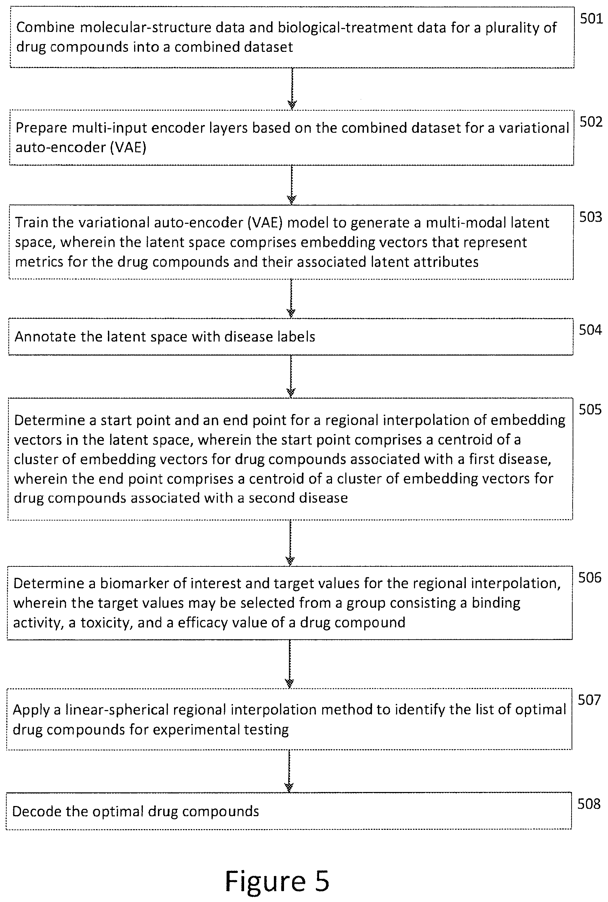

**Figure 5** illustrates an exemplary process implemented by a system **100** that may combine (block **501**) molecular-structure data **6** and biological-treatment data **7** for a plurality of drug compounds **3** into a combined dataset **5**, in accordance with certain embodiments. The system **100** may further prepare multi-input encoder layers **37** based on the combined dataset **5** for a variational auto-encoder **4** (block **502**). As depicted in block **503**, the system **100** may train the variational auto-encoder model **4** to generate a multi-modal latent space **1**, wherein the latent space **1** comprises embedding vectors **2** that represent probability distributions **10** for latent attributes **9** associated with the drug compounds **3**. The system **100** may also annotate (block **504**) the latent space **1** with disease labels **23**.

Further, the system **100** may determine a start point **38** and an end point **39** for a regional interpolation of embedding vectors **2** in the latent space **1** (block **505**). The start point **38** may comprise a centroid **22** of a cluster **20** of embedding vectors **2** for drug compounds **3** associated with a first disease **11**, and the end point **39** may comprise a centroid **22** of a cluster **20** of embedding vectors **2** for drug compounds **3** associated with a second disease **11**. In an embodiment, the first disease **11** may be associated with HIV and the second disease **11** may be associated with cancer.

The system **100** may determine a biomarker **12** of interest and target values **13** for the regional interpolation (**506**). The target values **13** may include a binding activity value, a toxicity value, and a efficacy value of a drug compound

3. Block **507** depicts the application of a linear-spherical regional interpolation method by the system **100** in order to identify the list of optimal drug compounds **3** for experimental testing. The system **100** may further decode (block **508**) the optimal drug compounds **3**.

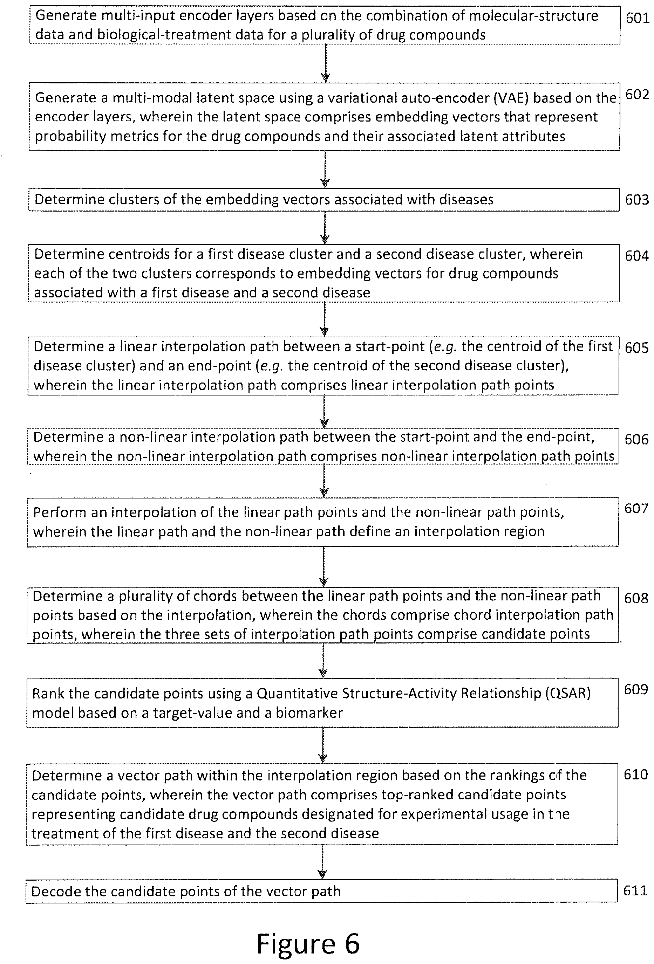

As illustrated in **Figure 6**, an embodiment of a system **100** may implement an exemplary process or method that generates (block **601**) multi-input encoder layers **37** based on the combination of molecular-structure data **6** and biological-treatment data **7** for a plurality of drug compounds **3**. At block **602**, the system **100** may generate a multi-modal latent space **1** using a variational auto-encoder **4** based on the encoder layers **37**. The latent space **1** may comprise embedding vectors **2** that represent probability distributions **10** for latent attributes **9** associated with the drug compounds **3**. The system **100** may further determine clusters **20** of the embedding vectors **2** associated with diseases **11** (block **603**), and determine centroids **22** for a first disease cluster **20** and a second disease cluster **20**. Each of the two clusters **20** may correspond to embedding vectors **2** for drug compounds **3** associated with a first disease **11** and a second disease **11** (block **604**).

As depicted in block **605**, the system **100** may determine a linear interpolation path **19** between a start-point **38** (*e.g.,* the centroid **22** of the first disease cluster **20**) and an end-point **39** (*e.g.,* the centroid **22** of the second disease cluster **20**). The linear interpolation path **19** may comprise linear interpolation path points **24**. In an embodiment, the linear interpolation path points **24** comprise the aforementioned first set of candidate points **24** on the linear interpolation path **19** that are based on the first predetermined stop-parameter **25**. Further, the system **100** may determine a non-linear interpolation path **21** between the start-point **38** and the end-point **39**, wherein the non-linear interpolation path **21** comprises non-linear interpolation path points **26** (block **606**). In an embodiment, the non-linear interpolation path points **26** comprise the aforementioned second set of candidate points **26** on the linear interpolation path **21** that are based on the first predetermined stop-parameter **25**.

As set forth in block **607**, the system **100** may perform an interpolation of the linear interpolation path points **24** and the non-linear interpolation path points **26**, wherein the linear interpolation path **19** and the non-linear interpolation path **21** define an interpolation region **31**. In addition, the system **100** may determine a plurality of chords **28** between the linear interpolation path points **24** and the corresponding non-linear interpolation path points **26** based on the interpolation (block **608**), wherein the chords **28** comprise a third set of candidate points **29**. The system **100** may rank (block **609**) these candidate points **29** using a Quantitative Structure-Activity Relationship (QSAR) model **33** based on a target-value **13** and a biomarker **12**. To provide additional context of the technical field and the QSAR model **33** disclosed herein, the contents of U.S. Patent No. 10,301/273, which issued on May 28, 2019, that describe QSAR methods are hereby referenced. Further, the system **100** may determine a vector path **35** within the interpolation region **31** based on the rankings of the candidate points **29** (block **610**). The vector path **35** may comprise embedding vectors **2** representing candidate drug compounds **3** designated for experimental usage in the treatment of the first disease **11** and the second disease **11**. The system **100** may also decode (block **611**) the embedding vectors **2** of the vector path **35**.

The interpolation implemented by embodiments of the disclosed systems and methods may include a linear interpolation (LERP) operation and a spherical linear interpolation (SLERP) operation. A number of intermediate points **24** along the linear interpolation path **19** may generated. Setting a parameter t equal to 10, the LERP interpolation may generate ten intermediate points **24** along the linear interpolation path **19** using the following function with multivariate input data **5** denoted as $v_0$ and $v_1$:

$$LERP(v_0, v_1, t) = v_0 + t(v_1 - v_0)$$

A number of intermediate points **26** may generated along a spherical interpolation path **21**. Setting a parameter t equal to 10, the SLERP interpolation may generate ten intermediate points **26** along the linear interpolation path **21** using the following function with multivariate input data **5** denoted as $v_0$ and $v_1$:

$$SLERP(v_0, v_1, t) = \frac{sin((1-t)\theta)}{sin\theta} v_0 + \frac{sin(t\theta)}{sin\theta} v_1$$

The SLERP path **21** is the spherical geometry equivalent of a path along the LERP path **19**. When the end vectors are perpendicular, the operation may comprise the parametric circle formula, in accordance with certain embodiments:

$$\vec{c} = (cos\theta)\hat{x} + (sin\theta)\hat{y} = (cos\theta)v_0 + (sin\theta)v_1$$

In another embodiment, the non-linear interpolation path **21** may elliptical. Such a non-linear interpolation path **21** may be generated using the following function, wherein each component may be scaled to the lengths of the semi-major and semi-minor axes of the ellipse, $\alpha$ and $\beta$ respectively:

$$\vec{e} = \alpha(cos\theta)\hat{x} + \beta(sin\theta)\hat{y} = \alpha(cos\theta)\, v_0 + \beta(sin\theta)\, v_1$$

While the present disclosure has been particularly shown and described with reference to an embodiment thereof, it will be understood by those skilled in the art that various changes in form and details may be made therein without departing from the scope as defined by the appended claims.

**Claims**

1.  A computer-implemented method for latent space exploration based on drug molecular-structure data and drug biological-treatment data to determine a drug compound for treating diseases, comprising the steps of:

    receiving drug molecular-structure data corresponding to a plurality of drug compounds;
    receiving drug biological-treatment data corresponding to the plurality of drug compounds;
    combining the received molecular-structure data and the received biological-treatment data into a combined dataset;
    generating multi-input encoder layers based on the combined dataset;
    generating a multi-modal latent space using a variational auto-encoder based on the encoder layers, wherein the latent space comprises embedding vectors, and wherein each embedding vector represents probability distributions for attributes of one of the plurality of drug compounds;
    determining clusters of the embedding vectors in the latent space associated with diseases, wherein determining clusters associated with diseases comprises performing a query of the embedding vectors in the latent space to identify the clusters, wherein the query is selected from a group consisting of a drug molecular-structure query, a drug treatment query, and a drug effect query;
    determining centroids for a first disease cluster and a second disease cluster, wherein each of the two clusters corresponds to embedding vectors for drug compounds associated with a first disease and a second disease;
    determining a linear interpolation path between a start-point and an end-point, wherein the start-point comprises the centroid of the first disease cluster, wherein the end-point comprises the centroid of the second disease cluster, wherein the linear interpolation path comprises linear interpolation path points, and wherein the linear interpolation path points comprise embedding vectors in the latent space;
    determining a non-linear interpolation path between the start-point and the end-point, wherein the non-linear interpolation path comprises non-linear interpolation path points, wherein the linear interpolation path and the non-linear interpolation path define an interpolation region, and wherein the non-linear interpolation path points comprise embedding vectors in the latent space;
    performing an interpolation of the linear interpolation path points and the non-linear interpolation path points;
    determining a plurality of chords between the linear interpolation path points and the non-linear interpolation path points based on the interpolation, wherein the chords comprise candidate points;
    ranking the candidate points using a quantitative structure-activity relationship model based on a target-value and a biomarker, wherein each of the ranked candidate points have a score determined by the quantitative structure-activity relationship model, wherein the candidate points are ranked based on the scores;
    determining a vector path within the interpolation region based on the rankings of the candidate points, wherein the vector path comprises the candidate points having the largest score on each chord and wherein the vector path comprises embedding vectors representing candidate drug compounds designated for experimental usage in the treatment of the first disease and the second disease; and,
    determine candidate drug molecular structures based on the candidate points by decoding the embedding vectors of the vector path wherein a drug molecular structure is determined for each candidate point of the vector path.

2.  The computer-implemented method of claim 1, wherein the molecular-structure data comprises simplified molecular-input line-entry system, SMILES, strings for the plurality of drug compounds.

3.  The computer-implemented method of claim 1 or claim 2, wherein the biological-treatment data is selected from a group consisting of: genetic variation data, somatic mutation data, electronic health records data, pathway enrichment data, gene expression data, protein expression data, disease ontology data, and protein interactions data; or wherein the biological-treatment data comprises genetic variation data, somatic mutation data, electronic health records data, pathway enrichment data, gene expression data, protein expression data, disease ontology data, and protein interactions data.

4. The computer-implemented method of any preceding claim, wherein the target-value is selected from a group consisting of: binding activity, toxicity, and efficacy; and/or
the non-linear interpolation path is selected from a group consisting of: a spherical path, a circular path, and an elliptical path.

5. The computer-implemented method of any preceding claim, wherein the step of determining clusters associated with diseases comprises the step of:
annotating the embedding vectors of the clusters with disease labels, wherein the disease labels correspond to the first disease and the second disease.

6. The method of any preceding claim, furthering comprising the steps of:

   determining a first plurality of candidate points on the linear interpolation path based on a first predetermined stop-parameter; and,
   determining a second plurality of candidate points on the non-linear interpolation path based on the first predetermined stop-parameter.

7. The method of claim 6, wherein determining a plurality of chords between the linear interpolation path points and the non-linear interpolation path points based on the interpolation comprises determining a linear chord path between candidate points on the linear interpolation path and corresponding candidate points on the non-linear interpolation path.

8. The method of claim 7, wherein the candidate points of the chords are a third plurality of candidate points, and the method further comprises the steps of:

   determining the third plurality of candidate points on each linear chord path based on a second predetermined stop-parameter; and,
   determining a drug effect score of each of the first plurality of candidate points, each of the second plurality of candidate points, and each of the third plurality of candidate points using a quantitative structure-activity relationship model.

9. The method of claim 8, furthering comprising the step of:
determining the candidate points based on the drug effect scores.

10. A product for latent space exploration based on drug molecular-structure data and drug biological-treatment data to determine a drug compound for treating diseases, comprising:

   a machine-readable medium, other than a transitory signal; and,
   instructions stored on the machine-readable medium, the instructions configured to, when executed, cause processing circuitry to perform the method of any one of claims 1-9.

11. A system for latent space exploration based on drug molecular-structure data and drug biological-treatment data to determine a drug compound for treating diseases, the system configured to perform the method according to any one of claims 1 - 9.

**Patentansprüche**

1. Computerimplementiertes Verfahren zur Erforschung eines latenten Raums basierend auf Daten der Molekularstruktur eines Arzneimittels und Daten der biologischen Aufbereitung eines Arzneimittels zum Bestimmen einer Arzneimittelverbindung zur Behandlung von Krankheiten, wobei das Verfahren die folgenden Schritte umfasst:

   Empfangen von Daten der Molekularstruktur eines Arzneimittels, die einer Vielzahl von Arzneimittelverbindungen entsprechen;
   Empfangen von Daten der biologischen Aufbereitung eines Arzneimittels, die einer Vielzahl von Arzneimittelverbindungen entsprechen;

Kombinieren der erhaltenen Daten der Molekularstruktur und der erhaltenen Daten der biologischen Aufbereitung in einem kombinierten Datensatz;

Erzeugen von Multi-Input-Encoder-Schichten basierend auf dem kombinierten Datensatz;

Erzeugen eines multimodalen latenten Raums unter Verwendung eines Variational Autoencoders basierend auf den Encoder-Schichten, wobei der latente Raum Einbettungsvektoren umfasst, und wobei jeder Einbettungsvektor Wahrscheinlichkeitsverteilungen für Merkmale einer der Vielzahl von Arzneimittelverbindungen repräsentiert;

Bestimmen von Anhäufungen der Einbettungsvektoren in dem latenten Raum, die mit Krankheiten verbunden sind, wobei das Bestimmen von Anhäufungen, die mit Krankheiten verbunden sind, die Durchführung einer Abfrage der Einbettungsvektoren in dem latenten Raum umfasst, um die Anhäufungen zu bestimmen, wobei die Abfrage aus einer Gruppe bestehend aus einer Abfrage der Molekularstruktur eines Arzneimittels, einer Abfrage einer Arzneimittelbehandlung, und einer Abfrage der Arzneimittelwirkung ausgewählt wird;

Bestimmen von Schwerpunkten für eine erste Krankheitsanhäufung und eine zweite Krankheitsanhäufung, wobei jede der zwei Anhäufungen Einbettungsvektoren für Arzneimittelverbindungen entspricht, die mit einer ersten Krankheit und einer zweiten Krankheit verbunden sind;

Bestimmen eines linearen Interpolationspfads zwischen einem Startpunkt und einem Endpunkt, wobei der Startpunkt den Schwerpunkt der ersten Krankheitsanhäufung umfasst, wobei der Endpunkt den Schwerpunkt der zweiten Krankheitsanhäufung umfasst, wobei der lineare Interpolationspfad lineare Interpolationspfad-Punkte umfasst, und wobei die linearen Interpolationspfad-Punkte Einbettungsvektoren in dem latenten Raum umfassen;

Bestimmen eines nichtlinearen Interpolationspfads zwischen dem Startpunkt und dem Endpunkt, wobei der nichtlineare Interpolationspfad nichtlineare Interpolationspfad-Punkte umfasst, wobei der lineare Interpolationspfad und der nichtlineare Interpolationspfad einen Interpolationsbereich festlegen, und wobei die nichtlinearen Interpolationspfad-Punkte Einbettungsvektoren in dem latenten Raum umfassen;

Ausführen einer Interpolation der linearen Interpolationspfad-Punkte und der nichtlinearen Interpolationspfad-Punkte;

Bestimmen einer Vielzahl von Akkorden zwischen den linearen Interpolationspfad-Punkten und den nichtlinearen Interpolationspfad-Punkten basierend auf der Interpolation, wobei die Akkorde Kandidatenpunkte umfassen;

Einstufen der Kandidatenpunkte unter Verwendung eines quantitativen Struktur-Wirkungs-Beziehungsmodells basierend auf einem Zielwert und einem Biomarker, wobei jeder der eingestuften Kandidatenpunkte ein Ergebnis aufweist, das durch das quantitative Struktur-Wirkungs-Beziehungsmodell bestimmt wird, wobei die Kandidatenpunkte basierend auf den Ergebnissen eingestuft werden;

Bestimmen eines Vektorpfads innerhalb des Interpolationsbereichs basierend auf den Einstufungen der Kandidatenpunkte, wobei der Vektorpfad die Kandidatenpunkte mit dem höchsten Ergebnis auf jedem Akkord umfasst, und wobei der Vektorpfad Einbettungsvektoren umfasst, die Kandidaten-Arzneimittelverbindungen darstellen, die zur experimentellen Verwendung bei der Behandlung der ersten Krankheit und der zweiten Krankheit bestimmt sind; und

Bestimmen von Kandidaten-Arzneimittel-Molekularstrukturen basierend auf den Kandidatenpunkten, indem die Einbettungsvektoren des Vektorpfads dekodiert werden, wobei eine molekulare Arzneimittelstruktur für jeden Kandidatenpunkt des Vektorpfads bestimmt wird.

2.  Computerimplementiertes Verfahren nach Anspruch 1, wobei die Daten der molekularen Struktur Strings eines Simplified Molecular Input Line Entry Systems, SMILES, für die Vielzahl von Arzneimittelverbindungen umfassen.

3.  Computerimplementiertes Verfahren nach Anspruch 1 oder Anspruch 2, wobei die Daten der biologischen Aufbereitung aus einer Gruppe ausgewählt werden, die besteht aus: Daten über genetische Variationen, Daten über somatische Mutationen, elektronische Gesundheitsdaten, Pfad-Anreicherungsdaten, Daten über Genexpression, Daten über Proteinexpression, Daten über die Krankheitsontologie und Daten über die Protein-Wechselwirkungen; oder

wobei die biologischen Aufbereitungsdaten Daten über genetische Variationen, Daten über eine somatische Mutation, elektronische Gesundheitsdaten, Pfad-Anreicherungsdaten, Daten über Genexpression, Daten über Proteinexpression, Daten über die Krankheitsontologie und Daten über die Protein-Wechselwirkungen umfassen.

4.  Computerimplementiertes Verfahren nach einem der vorhergehenden Ansprüche, wobei der Zielwert aus einer Gruppe ausgewählt wird, die besteht aus: Bindungsaktivität, Toxizität und Wirksamkeit; und/oder

der nichtlineare Interpolationspfad aus einer Gruppe ausgewählt wird, die besteht aus: sphärischem Pfad, Kreisbahn und/oder elliptischem Pfad.

5. Computerimplementiertes Verfahren nach einem der vorhergehenden Ansprüche, wobei der Schritt des Bestimmens von Anhäufungen, die mit Krankheiten verbunden sind, den folgenden Schritt umfasst:
Kommentieren der Einbettungsvektoren der Anhäufungen mit Krankheits-Bezeichnungen, wobei die Krankheitsbezeichnungen der ersten Krankheit und der zweiten Krankheit entsprechen.

6. Verfahren nach einem der vorhergehenden Ansprüche, das weiter die folgenden Schritte umfasst:

Bestimmen einer ersten Vielzahl von Kandidatenpunkten auf dem linearen Interpolationspfad basierend auf einem ersten vorher festgelegten Stopp-Parameter; und
Bestimmen einer zweiten Vielzahl von Kandidatenpunkten auf dem nichtlinearen Interpolationspfad basierend auf dem ersten vorher festgelegten Stopp-Parameter.

7. Verfahren nach Anspruch 6, wobei das Bestimmen einer Vielzahl von Akkorden zwischen dem linearen Interpolationspfad-Punkten und den nichtlinearen Interpolationspfad-Punkten basierend auf der Interpolation das Bestimmen eines linearen Akkordpfads zwischen Kandidatenpunkten auf dem linearen Interpolationspfad und entsprechenden Kandidatenpunkten auf dem nichtlinearen Interpolationspfad umfasst.

8. Verfahren nach Anspruch 7, wobei die Kandidatenpunkte der Akkorde eine dritte Vielzahl von Kandidatenpunkten sind, und das Verfahren weiter die folgenden Schritte umfasst:

Bestimmen der dritten Vielzahl von Kandidatenpunkten auf jedem linearen Akkordpfad basierend auf einem zweiten vorher festgelegten Stopp-Parameter; und
Bestimmen eines Ergebnisses der Arzneimittelwirkung eines jeden der ersten Vielzahl von Kandidatenpunkten, eines jeden der zweiten Vielzahl von Kandidatenpunkten und eines jeden der dritten Vielzahl von Kandidatenpunkten, indem ein quantitatives Struktur-Wirkungs-Beziehungsmodell verwendet wird.

9. Verfahren nach Anspruch 8, das weiter den folgenden Schritt umfasst:
Bestimmen der Kandidatenpunkte basierend auf den Arzneimittelwirkungs-Ergebnissen.

10. Produkt zur Erforschung eines latenten Raums basierend auf Daten der molekularen Struktur eines Arzneimittels und Daten der biologischen Aufbereitung eines Arzneimittels, um eine Arzneimittelverbindung zur Behandlung von Krankheiten zu bestimmen, wobei das Produkt umfasst:

einen maschinenlesbaren Datenträger, mit Ausnahme von einem vorübergehenden Signal; und
Befehle, die auf dem maschinenlesbaren Datenträger gespeichert sind, wobei die Befehle, sobald sie ausgeführt werden, konfiguriert sind, eine Verarbeitungsschaltung zum Ausführen des Verfahrens nach einem der Ansprüche 1 bis 9 zu veranlassen.

11. System zur Erforschung eines latenten Raums basierend auf Daten der molekularen Struktur eines Arzneimittels und Daten der biologischen Aufbereitung eines Arzneimittels, um eine Arzneimittelverbindung zur Behandlung von Krankheiten zu bestimmen, wobei das System zur Ausführung des Verfahrens nach einem der Ansprüche 1 bis 9 konfiguriert ist.

**Revendications**

1. Procédé mis en oeuvre par ordinateur pour l'exploration d'un espace latent sur la base de données sur la structure moléculaire d'un médicament et de données sur le traitement biologique médicamenteux afin de déterminer un composé médicamenteux permettant de traiter des maladies, comprenant les étapes consistant à :

recevoir des données sur la structure moléculaire du médicament correspondant à une pluralité de composés médicamenteux ;
recevoir des données sur le traitement biologique médicamenteux correspondant à la pluralité de composés médicamenteux ;
combiner les données sur la structure moléculaire reçues et les données sur le traitement biologique reçues en un ensemble de données combiné ;

générer des couches d'encodage multi-entrées sur la base de l'ensemble de données combiné ;

générer un espace latent multimodal, à l'aide d'un auto-encodeur variationnel, sur la base des couches d'encodage, dans lequel l'espace latent comprend des vecteurs d'intégration, et dans lequel chaque vecteur d'intégration représente des distributions de probabilité pour les attributs de l'un de la pluralité de composés médicamenteux ;

déterminer des grappes des vecteurs d'intégration dans l'espace latent associées à des maladies, dans laquelle l'étape de détermination des grappes associées aux maladies consiste à effectuer une requête sur les vecteurs d'intégration dans l'espace latent pour identifier les grappes, dans laquelle la requête est choisie dans un groupe constitué d'une requête sur la structure moléculaire du médicament, d'une requête sur le traitement médicamenteux et d'une requête sur l'effet médicamenteux ;

déterminer des centroïdes d'une première grappe de maladies et d'une seconde grappe de maladies, dans lesquelles chacune des deux grappes correspond à des vecteurs d'intégration de composés médicamenteux associés à une première maladie et à une seconde maladie ;

déterminer un chemin d'interpolation linéaire entre un point de départ et un point d'arrivée, dans lequel le point de départ comprend le centroïde de la première grappe de maladies, dans lequel le point d'arrivée comprend le centroïde de la seconde grappe de maladies, dans lequel le chemin d'interpolation linéaire comprend les points du chemin d'interpolation linéaire, et dans lesquels les points du chemin d'interpolation linéaire comprennent des vecteurs d'intégration dans l'espace latent ;

déterminer un chemin d'interpolation non linéaire entre le point de départ et le point d'arrivée, dans lequel le chemin d'interpolation non linéaire comprend les points du chemin d'interpolation non linéaire, dans lesquels le chemin d'interpolation linéaire et le chemin d'interpolation non linéaire définissent une région d'interpolation, et dans lesquels les points du chemin d'interpolation non linéaire comprennent des vecteurs d'intégration dans l'espace latent ;

effectuer une interpolation des points du chemin d'interpolation linéaire et des points du chemin d'interpolation non linéaire ;

déterminer une pluralité de cordes entre les points du chemin d'interpolation linéaire et les points du chemin d'interpolation non linéaire sur la base de l'interpolation, dans lesquelles les cordes comprennent des points candidats ;

classer les points candidats, à l'aide d'un modèle de relation quantitative structure-activité, sur la base d'une valeur cible et d'un biomarqueur, dans lesquels chacun des points candidats classés présente un score déterminé par le modèle de relation quantitative structure-activité, dans lesquels les points candidats sont classés sur la base des scores ;

déterminer un chemin vectoriel dans la région d'interpolation sur la base des classements des points candidats, dans lequel le chemin vectoriel comprend les points candidats ayant le score le plus élevé sur chaque corde, et dans lequel le chemin vectoriel comprend des vecteurs d'intégration représentant des composés médicamenteux candidats désignés pour une utilisation expérimentale dans le traitement de la première maladie et de la seconde maladie ; et,

déterminer les structures moléculaires des médicaments candidats sur la base des points candidats en décodant les vecteurs d'intégration du chemin vectoriel, dans laquelle la structure moléculaire d'un médicament est déterminée pour chaque point candidat du chemin vectoriel.

2. Procédé mis en oeuvre par ordinateur de la revendication 1, dans lequel les données sur la structure moléculaire comprennent des chaînes en langage SMILES, Simplified Molecular Input Line Entry Spécification, de la pluralité de composés médicamenteux.

3. Procédé mis en oeuvre par ordinateur de la revendication 1 ou 2, dans lequel les données sur le traitement biologique sont choisies dans un groupe constitué de : données de variation génétique, données de mutation somatique, données de dossiers médicaux électroniques, données d'enrichissement de voies, données d'expression génique, données d'expression protéique, données d'ontologie des maladies, et données d'interactions protéiques ; ou dans lequel les données sur le traitement biologique comprennent des données de variation génétique, des données de mutation somatique, des données de dossiers médicaux électroniques, des données d'enrichissement de voies, des données d'expression génique, des données d'expression protéique, des données d'ontologie des maladies et des données d'interactions protéiques.

4. Procédé mis en oeuvre par ordinateur de toute revendication précédente, dans lequel la valeur cible est choisie dans un groupe constitué de : l'activité de liaison, la toxicité et l'efficacité ; et/ou le chemin d'interpolation non linéaire est choisi dans un groupe constitué de : un chemin sphérique, un chemin circulaire et un chemin elliptique.

**5.** Procédé mis en oeuvre par ordinateur de toute revendication précédente, dans lequel l'étape de détermination des grappes associées aux maladies comprend l'étape consistant à :
annoter les vecteurs d'intégration des grappes avec des étiquettes de maladie, dans lesquelles les étiquettes de maladie correspondant à la première maladie et à la seconde maladie.

**6.** Procédé de toute revendication précédente, comprenant en outre les étapes consistant à :

déterminer une première pluralité de points candidats sur le chemin d'interpolation linéaire sur la base d'un premier paramètre d'arrêt prédéterminé ; et,
déterminer une deuxième pluralité de points candidats sur le chemin d'interpolation non linéaire sur la base du premier paramètre d'arrêt prédéterminé.

**7.** Procédé de la revendication 6, dans lequel l'étape de détermination d'une pluralité de cordes entre les points du chemin d'interpolation linéaire et les points du chemin d'interpolation non linéaire sur la base de l'interpolation consiste à déterminer un chemin de corde linéaire entre les points candidats sur le chemin d'interpolation linéaire et les points candidats correspondants sur le chemin d'interpolation non linéaire.

**8.** Procédé de la revendication 7, dans lequel les points candidats des cordes sont une troisième pluralité de points candidats, et le procédé comprend en outre les étapes consistant à :

déterminer la troisième pluralité de points candidats sur chaque chemin de corde linéaire sur la base d'un second paramètre d'arrêt prédéterminé ; et,
déterminer un score d'effets médicamenteux de chacun de la première pluralité de points candidats, de chacun de la deuxième pluralité de points candidats et de chacun de la troisième pluralité de points candidats, à l'aide d'un modèle de relation quantitative structure-activité.

**9.** Procédé de la revendication 8, comprenant en outre l'étape consistant à :
déterminer les points candidats sur la base des scores d'effets médicamenteux.

**10.** Produit pour l'exploration d'un espace latent sur la base de données sur la structure moléculaire d'un médicament et de données sur le traitement biologique médicamenteux afin de déterminer un composé médicamenteux permettant de traiter des maladies, comprenant :

un support lisible par machine, autre qu'un signal transitoire ; et,
des instructions stockées sur le support lisible par machine, les instructions étant configurées pour, lorsqu'elles sont exécutées, amener les circuits de traitement à exécuter le procédé de l'une quelconque des revendications 1 à 9.

**11.** Système pour l'exploration d'un espace latent sur la base de données sur la structure moléculaire d'un médicament et de données sur le traitement biologique médicamenteux afin de déterminer un composé médicamenteux permettant de traiter des maladies, le système étant configuré pour exécuter le procédé selon l'une quelconque des revendications 1 à 9.

Figure 1(a)

Figure 1(b)

Figure 1(c)

Figure 1(d)

Figure 1(e)

EP 3 723 095 B1

Figure 1(f)

Figure 1(g)

EP 3 723 095 B1

Data Reception Circuitry 110

Latent Space Generation Circuitry 120

Regional Interpolation Circuitry 130

Computation Circuitry 140

100

Figure 2

Figure 3

Generate multi-input encoder layers based on molecular-structure data and biological-treatment data for a plurality of drug compounds | 401

Train a variational auto-encoder (VAE) based on the encoder layers | 402

Generate a multi-modal latent space using the VAE, wherein the latent space comprises embedding vectors that represent metrics for the drug compounds and latent attributes associated with the drug compounds | 403

Annotate clusters of embedding vectors in the latent space with disease labels | 404

Determine centroids for a first disease cluster and a second disease cluster, wherein each of the two clusters corresponds to embedding vectors for drug compounds associated with a first disease and a second disease | 405

Perform regional interpolation using the centroid of the first disease cluster, the centroid of the second disease cluster, a target-value, and a biomarker | 406

Decode embedding vectors determined by the regional interpolation and a quantitative structure activity relationship (QSAR) model, wherein the embedding vectors represent candidate drug compounds likely to have desired attributes for treating the first and second disease | 407

# Figure 4

Combine molecular-structure data and biological-treatment data for a plurality of drug compounds into a combined dataset | 501

Prepare multi-input encoder layers based on the combined dataset for a variational auto-encoder (VAE) | 502

Train the variational auto-encoder (VAE) model to generate a multi-modal latent space, wherein the latent space comprises embedding vectors that represent metrics for the drug compounds and their associated latent attributes | 503

Annotate the latent space with disease labels | 504

Determine a start point and an end point for a regional interpolation of embedding vectors in the latent space, wherein the start point comprises a centroid of a cluster of embedding vectors for drug compounds associated with a first disease, wherein the end point comprises a centroid of a cluster of embedding vectors for drug compounds associated with a second disease | 505

Determine a biomarker of interest and target values for the regional interpolation, wherein the target values may be selected from a group consisting a binding activity, a toxicity, and a efficacy value of a drug compound | 506

Apply a linear-spherical regional interpolation method to identify the list of optimal drug compounds for experimental testing | 507

Decode the optimal drug compounds | 508

# Figure 5

Generate multi-input encoder layers based on the combination of molecular-structure data and biological-treatment data for a plurality of drug compounds | 601

Generate a multi-modal latent space using a variational auto-encoder (VAE) based on the encoder layers, wherein the latent space comprises embedding vectors that represent probability metrics for the drug compounds and their associated latent attributes | 602

Determine clusters of the embedding vectors associated with diseases | 603

Determine centroids for a first disease cluster and a second disease cluster, wherein each of the two clusters corresponds to embedding vectors for drug compounds associated with a first disease and a second disease | 604

Determine a linear interpolation path between a start-point (*e.g.* the centroid of the first disease cluster) and an end-point (*e.g.* the centroid of the second disease cluster), wherein the linear interpolation path comprises linear interpolation path points | 605

Determine a non-linear interpolation path between the start-point and the end-point, wherein the non-linear interpolation path comprises non-linear interpolation path points | 606

Perform an interpolation of the linear path points and the non-linear path points, wherein the linear path and the non-linear path define an interpolation region | 607

Determine a plurality of chords between the linear path points and the non-linear path points based on the interpolation, wherein the chords comprise chord interpolation path points, wherein the three sets of interpolation path points comprise candidate points | 608

Rank the candidate points using a Quantitative Structure-Activity Relationship (QSAR) model based on a target-value and a biomarker | 609

Determine a vector path within the interpolation region based on the rankings of the candidate points, wherein the vector path comprises top-ranked candidate points representing candidate drug compounds designated for experimental usage in the treatment of the first disease and the second disease | 610

Decode the candidate points of the vector path | 611

# Figure 6

## REFERENCES CITED IN THE DESCRIPTION

**Patent documents cited in the description**

- US 10301273 B

**Non-patent literature cited in the description**

- **RAFAEL GOMEZ-BOMBARELLI.** *Automatic chemical design using a data-driven continuous representation of molecules*